# EUROPÄISCHE PATENTANMELDUNG

(11) **EP 2 082 700 A1**
(43) Veröffentlichungstag der Anmeldung: **29.07.2009**
(21) Anmeldenummer: 08007194.7
(22) Anmeldetag: 11.04.2008
(51) Int. Cl.: A61B 19/00, G02B 7/00, G02B 21/00, A61B 17/00

(54) **Anordnung aus einem Operationsmikroskop und einer Anzeigeeinheit**

(30) Priorität: 24.01.2008 DE 202008001363 U; 24.01.2008 DE 202008001540 U
(71) Anmelder: Möller-Wedel GmbH, 22880 Wedel (DE)
(72) Erfinder: Schmidt, Martin, Dr., 23611 Bad Schwartau (DE); Schalt, Peter, 25436 Moorrege (DE)
(74) Vertreter: Glawe, Delfs, Moll

(57) **Zusammenfassung**

Die Erfindung betrifft eine Anordnung aus einem Operationsmikroskop (11) und einer Anzeigeeinheit (17). Das Operationsmikroskop (11) umfasst ein Okular (15). Die Anzeigeeinheit (17) umfasst einen Bildschirm (18) und ist dazu ausgelegt ist, externe Informationen von einer Informationsquelle (20) zu empfangen und auf dem Bildschirm (18) anzuzeigen. Erfindungsgemäß ist der Bildschirm (18) in einem horizontalen Abstand zu einer sich durch das Okular (15) erstreckenden vertikalen Achse (9) angeordnet ist, der nicht größer ist als 90 cm, vorzugsweise nicht größer als 50 cm, weiter vorzugsweise nicht größer als 30 cm. Dies ermöglicht es dem Chirurgen, der von dem Operationsmikroskop aufblickt, die Informationen auf dem Bildschirm wahrzunehmen, ohne dass seine Augen sich auf eine andere Sehentfernung einstellen müssen.

## Beschreibung

Die Erfindung betrifft eine Anordnung aus einem Operationsmikroskop und einer Anzeigeeinheit. Das Operationsmikroskop umfasst ein Okular. Die Anzeigeeinheit umfasst einen Bildschirm und ist dazu ausgelegt, externe Informationen von einer Informationsquelle zu empfangen und auf dem Bildschirm anzuzeigen.

Bei mikrochirurgischen Operationen betrachtet der Chirurg das Operationsfeld durch ein Operationsmikroskop. Bei komplizierten Operationen benötigt der Chirurg zusätzliche Informationen. Beispielsweise können dies radiologische Bilder sein, die bei der Vorbereitung der Operation erstellt worden sind. Früher wurden physische Abzüge dieser radiologischen Bilder erstellt und in der Nähe des Operationsfelds aufgehängt, so dass der Chirurg sie bei Bedarf während der Operation betrachten konnte. Heute ist es in den meisten Fällen einfacher und günstiger, die Informationen auf einem Bildschirm anzuzeigen.

Wenn der Chirurg während der Operation zusätzliche Informationen benötigt, hebt er den Blick vom Operationsmikroskop und stellt seine Wahrnehmung um vom Mikroskopbild auf seine Umgebung im Operationssaal. Er tritt zu dem Bildschirm, nimmt die gewünschten Informationen auf und kehrt zum Operationsmikroskop zurück. Er richtet seinen Blick wieder durch das Operationsmikroskop auf das Operationsfeld und muss dabei erneut seine Wahrnehmung umstellen, dieses Mal von seiner Umgebung im Operationssaal auf das Mikroskopbild. Durch das Umstellen der Wahrnehmung zwischen dem Mikroskopbild und der Umgebung im Operationssaal geht wertvolle Zeit verloren, in der der Chirurg sich nicht auf die Operation konzentrieren kann.

Der Erfindung liegt die Aufgabe zu Grunde, eine Anordnung der eingangs genannten Art vorzustellen, mit der die Arbeit des Chirurgen erleichtert wird. Die Aufgabe wird gelöst durch die Merkmale des Anspruchs 1. Erfindungsgemäß ist der Bildschirm in einem horizontalen Abstand zu einer sich durch das Okular erstreckenden vertikalen Achse angeordnet, der nicht größer ist als 90 cm, vorzugsweise nicht größer als 50 cm, weiter vorzugsweise nicht größer als 30 cm. Vorteilhafte Ausführungsformen finden sich in den Unteransprüchen.

Zunächst werden einige Begriffe erläutert. Eine Information ist dann extern, wenn sie von dem Operationsmikroskop unabhängig ist. Die externen Informationen sind vor oder während der Operation in der Informationsquelle bereitgestellt worden. Zu den externen Informationen können Bilder gehören, die vor der Operation mit dem Operationsmikroskop aufgenommen worden sind und in der Zwischenzeit in der Informationsquelle gespeichert worden sind. Keine externe Information sind das aktuelle Bild des Operationsmikroskops sowie Daten über den Betriebszustand des Operationsmikroskops.

Der Begriff Information ist weit zu verstehen. Er umfasst jede Art von Bild, grafischer Darstellung, Text, Zahlen, Symbolen und sonstigen Daten, die bei einer Operation nützlich sein können.

Beim Blick durch das Operationsmikroskop sieht der Chirurg ein Mikroskopbild, das einen bestimmten scheinbaren Abstand von seinen Augen hat. Dieser scheinbare Abstand, auf den sich das Auge des Chirurgen beim Blick durch das Operationsmikroskop einstellt, wird im Rahmen der Erfindung als Sehentfernung bezeichnet. In Operationsmikroskopen liegt die Sehentfernung meist zwischen 20 cm und 30 cm. Erfindungsgemäß soll dem Chirurgen seine Arbeit dadurch erleichtert werden, dass er seinen Blick zwischen dem Operationsmikroskop und dem Bildschirm wechseln kann, ohne dass sich das Auge auf eine neue Sehentfernung einstellen muss.

Wenn der Chirurg vom Okular aufblickt, so hebt er normalerweise den Kopf etwas nach oben, ohne dass sich jedoch seine Augen deutlich von der vertikalen Achse entfernen, die sich durch das Okular erstreckt. Idealerweise ist der Bildschirm deswegen in einem horizontalen Abstand von der vertikalen Achse angeordnet, der der Sehentfernung entspricht, also in einem horizontalen Abstand zwischen 20 cm und 30 cm. Es hat sich jedoch gezeigt, dass der erfindungsgemäße Erfolg weitgehend auch dann noch erreicht wird, wenn der horizontale Abstand des Bildschirms zu der vertikalen Achse nicht exakt der Sehentfernung entspricht. Wenn der Chirurg nämlich beim Aufblicken vom Okular seine Augen leicht in Richtung des Bildschirms bewegt und zugleich seine Augen auf eine leicht veränderte Sehentfernung einstellt, so kann er auf einem bis zu 90 cm von der vertikalen Achse entfernten Bildschirm Informationen wahrnehmen, ohne dass eine besondere Konzentration erforderlich. Vorzuziehen ist es jedoch, wenn der Bildschirm in einem horizontalen Abstand von nicht mehr als 50 cm von der vertikalen Achse angeordnet ist.

Der Bildschirm sollte ferner auf Augenhöhe angeordnet sein, wenn der Chirurg seinen Blick in einer natürlichen Bewegung vom Okular hebt. Normalerweise erreicht man dies, indem man den Bildschirm oberhalb des Okulars anordnet, so dass der vertikale Abstand zwischen dem Okular und dem Bildschirm zwischen 1 cm und 50 cm, vorzugsweise zwischen 5 cm und 30 cm beträgt.

Wenn der Bildschirm seitlich von dem Okular angeordnet ist, muss der Chirurg den Kopf drehen, bevor er die Informationen von dem Bildschirm aufnehmen kann. Bequemer für den Chirurgen ist es, wenn er lediglich den Kopf von dem Okular heben muss und sein Blick dann unmittelbar auf den Bildschirm gerichtet ist. Dies wird erreicht, wenn der Bildschirm hinter dem Okular, also vom Chirurg aus gesehen auf der gegenüberliegenden Seite des Okulars, angeordnet ist.

Der Chirurg kann die Informationen auf dem Bildschirm am besten wahrnehmen, wenn der Bildschirm in Richtung des Chirurgen ausgerichtet ist. Gelegentlich ist es jedoch erwünscht, dass außer dem Chirurgen auch ein Assistent die Informationen auf dem Bildschirm wahrnehmen kann. Dazu kann der Bildschirm leicht zur Seite gedreht sein. Vorzugsweise ist eine im wesentlichen senkrechte Achse vorgesehen, um die der Bildschirm gedreht werden kann, um die Ausrichtung des Bildschirms zu verändern. Wenn der Chirurg gerade keine Informationen von dem Bildschirm benötigt, kann der Bildschirm um diese Achse soweit gedreht werden, dass er in die Richtung eines seitlich von oder hinter dem Operationsmikroskop stehenden Assistenten ausgerichtet ist. Um Reflexionen zu vermindern, kann der Bildschirm um eine im wesentlichen horizontale Achse schwenkbar sein.

Die Anzeigeeinheit, die dem Bildschirm umfasst, kann auf einem eigenen Gestell angebracht sein, das in die passende Position zu dem Operationsmikroskop gebracht wird. Übersichtlicher wird die Operationsumgebung jedoch, wenn die Anzeigeeinheit an der Aufhängung des Operationsmikroskops befestigt ist. Der Bildschirm bleibt dann automatisch in der richtigen Position relativ zu dem Okular, wenn die Position des Operationsmikroskops geändert wird. Die Befestigung zwischen der Anzeigeeinheiten und der Aufhängung des Operationsmikroskops kann gelenkig oder flexibel sein, so dass jeder Chirurg den Bildschirm in die für sich passende Position bringen kann.

Von der Erfindung umfasst ist es, dass zwischen der Informationsquelle und dem Bildschirm zu übertragende Informationen von einer dritten Person ausgewählt werden. Die dritte Person kann beispielsweise ein Assistent sein, der die Operation zusammen mit dem Chirurgen vorbereitet hat und der weiß, welche Informationen zu welchem Zeitpunkt benötigt werden. Die Informationsübertragung kann in diesem Fall beispielsweise von einem Nebenzimmer aus gesteuert werden. Insbesondere wird dies in Betracht kommen, wenn die während der Operation erforderlichen Informationen so umfangreich sind, dass der Chirurg dies nicht nebenbei übernehmen kann.

In einfacheren Fällen kann es besser sein, wenn der Chirurg selbst sich um die Datenübertragung zwischen der Informationsquelle und der Anzeigeeinheit kümmert. Dazu kann die Anzeigeeinheiten ein Bedienelement umfassen, mittels dessen der Chirurg die Informationen auswählen kann, die von der Informationsquelle zum Bildschirm übertragen werden sollen. Das Bedienelement kann in der unmittelbaren Umgebung des Bildschirms angeordnet sein. Möglich ist es auch, dass der Bildschirm ein so genannter Touchscreen ist, dass also das Bedienelement in den Bildschirm integriert ist und auf Berührung reagiert. Die Bedienung kann über ein auf dem Bildschirm angezeigtes Menü erfolgen.

In einer vorteilhaften Ausführungsform umfasst die Anzeigeeinheit ein Steuerelement, das in ähnlicher Weise ausgebildet sein kann wie das Bedienelement. Mit dem Steuerelement kann eine Funktion in der Umgebung des Operationsmikroskops gesteuert werden. Beispielsweise kann über das Steuerelement das Licht in dem Operationssaal angeschaltet oder ausgeschaltet werden, oder es können die Parameter eines für die Operation verwendeten Geräts verändert werden. Umgekehrt ist es möglich, Parameter des Geräts auf dem Bildschirm anzuzeigen.

Das aktuelle Mikroskopbild gehört nicht zu den externen Informationen im Sinne der Erfindung. Gleichwohl kann die Anzeigeeinheit so ausgebildet sein, dass in Zeitspannen, in denen keine externe Information für die Operation benötigt wird, auch das aktuelle Mikroskopbild auf dem Bildschirm angezeigt werden kann. Es kann beispielsweise zu Ausbildungszwecken hilfreich sein, wenn ein Assistent auf dem Bildschirm das gleiche Bild sehen kann, das auch der Chirurg durch das Mikroskop sieht.

Wenn eine Datenübertragung auch von der Anzeigeeinheit zu einem Speicher vorgesehen ist, können während der Operation aufgenommene Mikroskopbilder gespeichert und später ausgewertet werden. Außerdem ist es möglich, die Mikroskopbilder zu einem erfahrenen Chirurgen zu übertragen, der den Verlauf der Operation überwachen kann, ohne dass seine Anwesenheit im Operationssaal erforderlich ist. Wenn ihm etwas auffällt, kann er Ratschläge geben, die zu dem Bildschirm übertragen werden und die für den operierenden Chirurgen sichtbar sind. Bei Bedarf kann auch eine Sprachverbindung zwischen dem operierenden Chirurgen und dem überwachenden Chirurgen hergestellt werden.

Ferner kann das Operationsmikroskop eine Einspiegelungseinheit umfassen, mittels derer Informationen dem Mikroskopbild überlagert werden. Die Einspiegelungseinheit kann alternativ so eingestellt werden, dass die Informationen an statt des Mikroskopbilds oder zusätzlich zu dem Mikroskopbild dargestellt werden. Es kann vorgesehen sein, dass auf dem Bildschirm dargestellte Informationen zu der Einspiegelungseinheit übertragen werden können. Der Chirurg kann über ein Bedienelement der Anzeigeeinheit bestimmte Informationen für die Einspiegelungseinheit auswählen.

Im Operationssaal befindliche Gegenstände müssen steril sein. Bei dem Operationsmikroskop wird die Sterilität üblicherweise dadurch erreicht, dass das Operationsmikroskop steril abgedeckt wird. Auf die gleiche Weise kann auch eine Sterilität der Anzeigeeinheit erreicht werden. Wenn die sterile Abdeckung durchsichtig ist und glatt über den Bildschirm gespannt ist, bleiben die auf dem Bildschirm dargestellten Informationen trotzdem sichtbar. Besser können die Informationen auf dem Bildschirm jedoch wahrgenommen werden, wenn der Bildschirm von der sterilen Abdeckung ausgenommen ist. Um den Bildschirm von der sterilen Abdeckung auszunehmen, kann ein Rahmen vorgesehen sein, der den Teil der sterilen Abdeckung, der den Bildschirm bedeckt, ausschneidet, wenn er auf den Bildschirm aufgesteckt wird. Die Bedienelemente auf dem Bildschirm können mit einem sterilen Stift bedient werden.

Die Anzeigeeinheit verdient gegebenenfalls selbstständig Schutz, auch ohne dass sie mit dem Operationsmikroskop kombiniert ist. Der zentrale Aspekt der selbstständig erfinderischen Anzeigeeinheit kann darin bestehen, dass Informationen von einer externen Informationsquelle auf dem Bildschirm angezeigt werden. Alternativ kann der erfinderische Aspekt darin bestehen, dass die Anzeigeeinheit ein Steuerelement umfasst, das dazu ausgelegt ist, eine Funktion in der Umgebung der Anzeigeeinheit steuern.

Die Erfindung wird nachfolgend unter Bezugnahme auf die beigefügten Zeichnungen anhand vorteilhafter Ausführungsformen beispielhaft beschrieben. Es zeigen:
- Fig. 1: eine Ausführungsform einer erfindungsgemäßen Anordnung;
- Fig. 2: eine erste Ausführungsform eines erfindungsgemäßen Bildschirms;
- Fig. 3: eine zweite Ausführungsform eines erfindungsgemäßen Bildschirms; und
- Fig. 4: eine Ausführungsform einer erfindungsgemäßen Anzeigeeinheit;

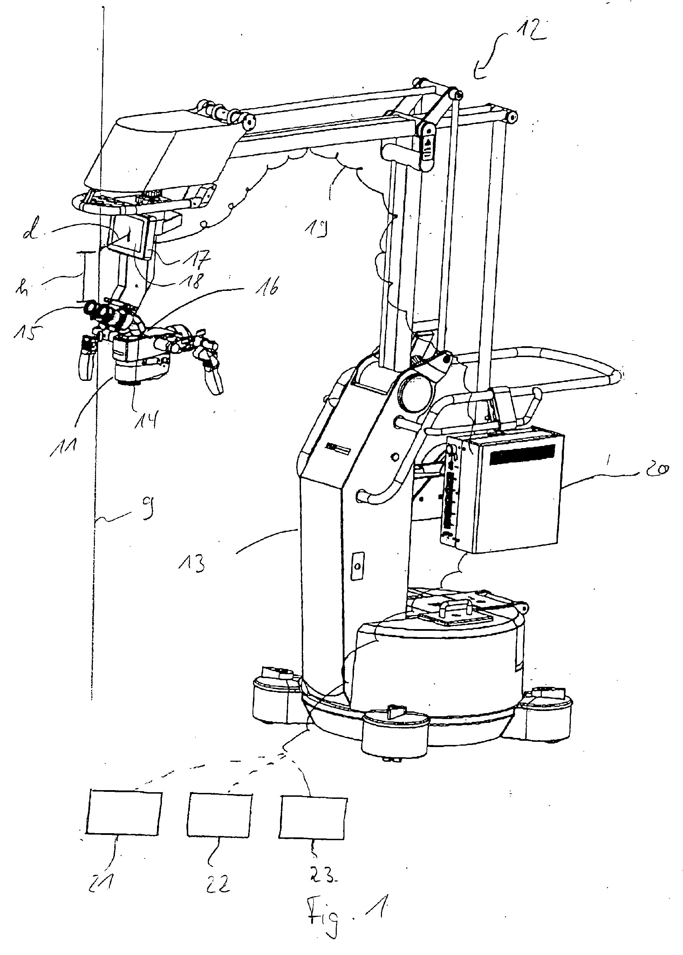

Fig. 1 zeigt ein Operationsmikroskop 11, das über eine gelenkige Aufhängung 12 an einem Stativ 13 befestigt ist. Über die gelenkige Aufhängung 12 kann das Operationsmikroskop 11 so in Position gebracht werden, dass der Chirurg in einer bequemen Haltung auf ein nicht dargestelltes Operationsfeld blicken kann. Das Operationsmikroskop 11 umfasst ein Objektiv 14 sowie ein Okular 15 mit Einblicken für beide Augen. Über eine Einspiegelungseinheit 16 können Informationen in das Operationsmikroskop 11 eingespiegelt werden.

An der gelenkigen Aufhängung 12 ist eine Anzeigeeinheit 17 angeordnet, die einen Bildschirm 18 umfasst. Der Bildschirm 18 ist in einem vertikalen Abstand h von ungefähr 20 cm oberhalb des Okulars 15 angeordnet. Er ist ferner in einem horizontalen Abstand d von ungefähr 30 cm zu einer vertikalen Achse 9 angeordnet, die sich durch das Okular 15 erstreckt. Der Chirurg, der von dem Okular 15 aufblickt und sich dabei leicht aufrichtet, kann auf dem Bildschirm 18 dargestellte Informationen wahrnehmen, ohne dass seine Augen sich auf eine andere Sehentfernung einstellen müssen. Die Anzeigeeinheit 17 ist um eine vertikale Achse um 360° drehbar, so dass ein Assistent den Bildschirm 18 in eine für ihn bequeme Position bringen kann. Ferner ist die Anzeigeeinheit 17 in geringerem Umfang auch um eine horizontale Achse schwenkbar, so dass Reflexionen vermieden werden können.

Die Anzeigeeinheit 17 ist über ein Kabel 19 mit einem Computer 20 als Informationsquelle verbunden. In dem Computer 20 sind Informationen gespeichert. Die Informationen können beispielsweise radiologische Bilder, bei der Planung der Operation erstellte Skizzen und andere für die Operation relevante Daten umfassen. Die Informationen können über das Kabel 19 zu der Anzeigeeinheit 17 übertragen und auf dem Bildschirm 18 angezeigt werden. Alternativ kann die Verbindung zwischen dem Computer 20 und der Anzeigeeinheit 17 auch drahtlos ausgeführt sein.

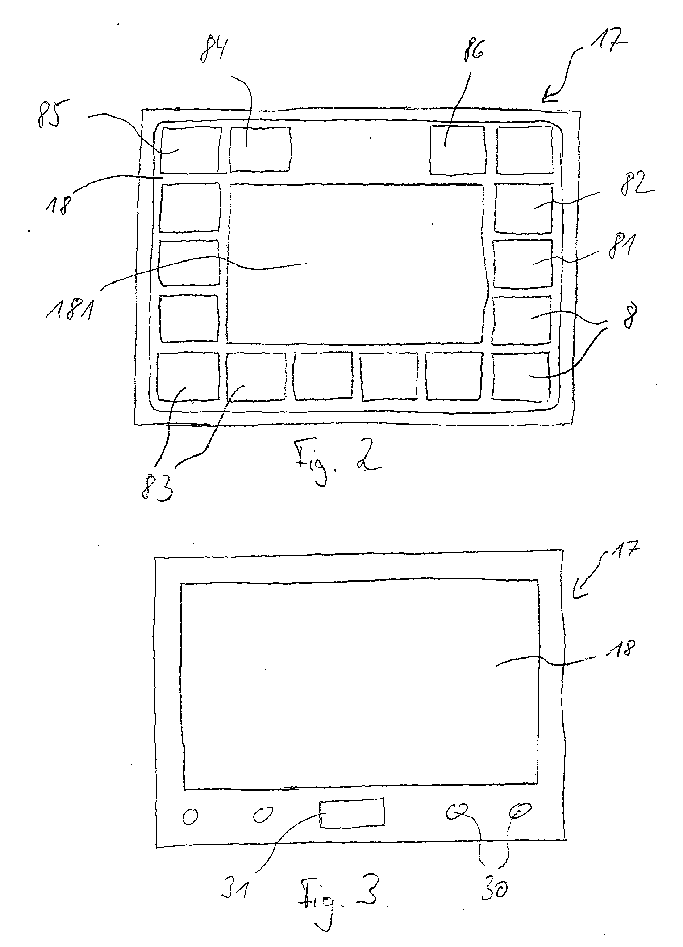

Die in Fig. 2 vergrößert dargestellte Anzeigeeinheit 17 umfasst einen Bildschirm 18, der als Touchscreen mit einer Mehrzahl von Bedienfeldern 8 ausgeführt ist. Durch Betätigen eines Bedienfelds 8 kann eine Information aus dem Computer 20 ausgewählt werden, die zu der Anzeigeeinheit 17 übertragen wird und im Zentralbereich 181 des Bildschirms 18 angezeigt wird. Durch Antippen des Zentralbereichs 181 kann der Bildschirm 18 in einem Vollbildmodus gebracht werden, in dem die Bedienfelder 8 nicht mehr sichtbar sind. Bei erneutem Antippen des Bildschirms 18 erscheinen die Bedienfelder 8 wieder.

Es ist möglich, einem einzelnen Bedienfeld 81 ein bestimmtes Bild aus dem Speicher des Computers 20 zuzuordnen. Es reichte dann ein Antippen des Bedienfelds 81, um dieses Bild auf dem Bildschirm 18 darzustellen. Die Bedienfelder 8 können auch mit wechselnden Funktionen belegt werden, beispielsweise wenn die Bedienfelder 8 Bestandteil einer Menüführung sind.

Der Computer 20 kann mit weiteren Informationsquellen verbunden sein. Diese Informationsquellen können beispielsweise nicht dargestellte Datenbanken wie das Bildarchiv des Krankenhauses oder ein Internetserver sein. Die Informationsquellen können aber auch Geräte 21, 22, 23 des Operationssaales seien, wie beispielsweise Sauger, Dissektoren, Bohrer, Koagulatoren, Endoskope, Ultraschallgeräte, Anästhesiegeräte, Geräte für das Patienten-Monitoring, Navigationssysteme, Videokameras, Phakoemulsifikationsgeräte, Vitrektomiegeräte, Endoilluminatoren, Kameras für die Fluoreszenz von ICG- oder ALA-Farbstoffen. Die von den Geräten 21, 22, 23 erhaltenen Informationen können beispielsweise in Informationen über den Betriebszustand oder sonstigen Parametern bestehen. Die Verbindung zu den Geräten kann über etablierte Standards wie LAN, W-LAN oder andere Netzwerke, beispielsweise mittels Video-, RS-232-, USB- oder Bluetooth-Anschlüssen erfolgen. Die Geräte können über IP-Adressen aufgerufen werden. In der Anzeigeeinheit 17 kann ein Bedienfeld 82 vorgesehen sein, über das ein bestimmtes Geräts unmittelbar aufgerufen werden kann. Das Bedienfeld 82 kann auch so programmiert sein, dass es zu einer Liste von IP-Adressen führt.

Auf dem Bildschirm 18 sind Bedienfelder 83 vorgesehen, die so programmiert sind, dass sie eine Steuerung von Funktionen der Geräte 21, 22, 23 ermöglichen. So können beispielsweise Sauger, Dissketoren, Bohrer und Koagulatoren eingeschaltet und ausgeschaltet werden, bei Endoskopen die Vergrößerung verändert werden und bei einem Ultraschallgerät eine neue Aufnahme ausgelöst werden. Ein weiteres Bedienfeld 84 kann dazu ausgelegt sein, den Bildschirm 18 abzudunkeln. Ein Bedienfeld 85 stellt eine Verbindung zu einer Internetseite her, auf der ein Gehirnatlas hinterlegt ist. Eine Schaltfläche 86 stellt eine Verbindung zur persönlichen Homepage des Chirurgen her, auf der er vor der Operation Informationen zur Operation hinterlegt hat.

Über eine drahtlose Kommunikationsverbindung können Informationen von der Anzeigeeinheit 17 zu der Einspiegelungseinheit 16 übertragen werden. Dies ist insbesondere dann von Nutzen, wenn es sich um einfache Informationen wie Zahlen oder Zeiger handelt, die dann gleichzeitig mit dem Mikroskopbild gesehen werden können, ohne die Augen von dem Okular zu entfernen. Die Einspiegelung kann stereoskopisch erfolgen.

Eine alternative Ausführungsform einer Anzeigeeinheit 17 ist in Fig. 3 gezeigt. Der Bildschirm 18 ist in diesem Fall ein autostereoskopischer 3D-Bildschirm. Alternativ ist der Bildschirm 18 ein polarisierter Bildschirm in Verbindung mit passiven oder aktiven Polarisationsbrillen. Der Bildschirm 18 ist nicht als Touchscreen ausgebildet, sondern die Bedienung erfolgt über Bedienelemente 30, die am Rahmen der Anzeigeeinheit 17 angeordnet sind. Eines der Bedienelemente 30 ist ein Touchpad 31, über das ein auf dem Bildschirm 18 angezeigtes Menü gesteuert werden kann.

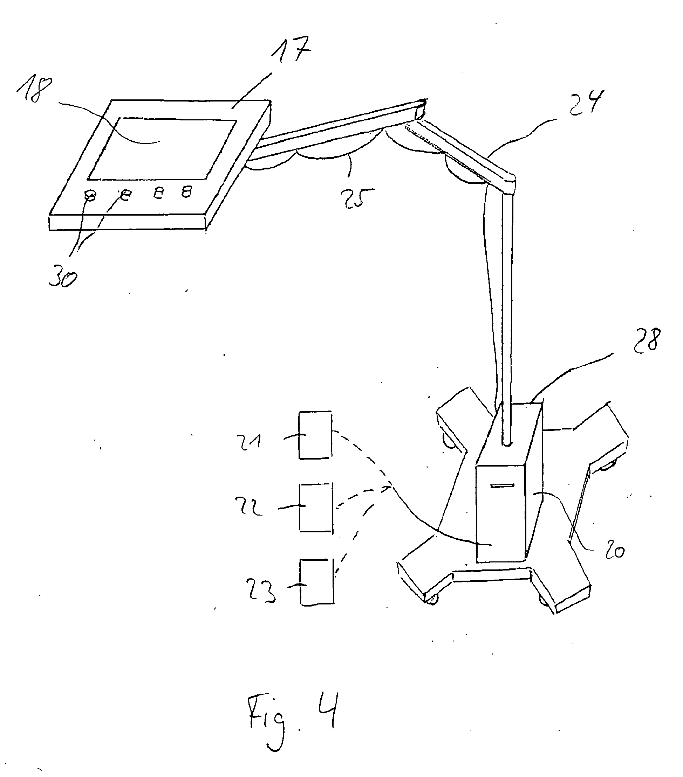

In Fig. 4 ist eine Anzeigeeinheit 17 gezeigt, die nicht zusammen mit dem Operationsmikroskop aufgehängt ist, sondern auf einem eigenen Stativ 28 ruht. Die Anzeigeeinheit 17 ist über eine gelenkige Aufhängung 24 mit dem Stativ 28 verbunden. Durch die gelenkige Aufhängung ist es möglich, die Anzeigeeinheit 17 mit dem Bildschirm 18 in eine passende Position relativ zu dem Okular eines Operationsmikroskops zu bringen.

Die Anzeigeeinheit 17 ist über ein Kabel 25 mit einem Computer 20 verbunden, der in das Stativ 28 integriert ist. Der Computer 20 wiederum ist mit Geräten 21, 22, 23 verbunden.

## Patentansprüche

1. Anordnung aus einem Operationsmikroskop (11) und einer Anzeigeeinheit (17), wobei das Operationsmikroskop (11) ein Okular (15) umfasst und wobei die Anzeigeeinheit (17) einen Bildschirm (18) umfasst und dazu ausgelegt ist, externe Informationen von einer Informationsquelle (20) zu empfangen und auf dem Bildschirm (18) anzuzeigen, **dadurch gekennzeichnet, dass** der Bildschirm (18) in einem horizontalen Abstand (d) zu einer sich durch das Okular (15) erstreckenden vertikalen Achse (9) angeordnet ist, der nicht größer ist als 90 cm, vorzugsweise nicht größer als 50 cm, weiter vorzugsweise nicht größer als 30 cm.

2. Anordnung nach Anspruch 1, **dadurch gekennzeichnet, dass** der Bildschirm (18) oberhalb des Okulars (15) angeordnet ist und dass der vertikale Abstand (h) zwischen dem Okular (15) und dem Bildschirm (18) zwischen 1 cm und 50 cm, vorzugsweise zwischen 5 cm und 30 cm beträgt.

3. Anordnung nach Anspruch 1 oder 2, **dadurch gekennzeichnet, dass** der Bildschirm (18) hinter dem Okular (15) angeordnet ist.

4. Anordnung nach einem der Ansprüche 1 bis 3, **dadurch gekennzeichnet, dass** die Ausrichtung des Bildschirms (18) veränderbar ist.

5. Anordnung nach einem der Ansprüche 1 bis 4, **dadurch gekennzeichnet, dass** die Anzeigeeinheit (17) an der Aufhängung (12) des Operationsmikroskops (11) befestigt ist.

6. Anordnung nach einem der Ansprüche 1 bis 5, **dadurch gekennzeichnet, dass** die Anzeigeeinheit (17) ein Bedienelement (8) umfasst, das dazu ausgelegt ist, die von der Informationsquelle (20) zum Bildschirm (18) übertragenen Informationen auszuwählen.

7. Anordnung nach Anspruch 6, **dadurch gekennzeichnet, dass** das Bedienelement (8) in den Bildschirm (18) integriert ist und auf Berührung reagiert.

8. Anordnung nach einem der Ansprüche 1 bis 7, **dadurch gekennzeichnet, dass** die Anzeigeeinheit (17) ein Steuerelement (83) umfasst, das dazu ausgelegt ist, eine Funktion in der Umgebung des Operationsmikroskops (11) zu steuern.

9. Anordnung nach einem der Ansprüche 1 bis 8, **dadurch gekennzeichnet, dass** die Anzeigeeinheit (17) einen Modus umfasst, in dem das durch das Operationsmikroskop (11) sichtbare Bild auf dem Bildschirm (18) angezeigt wird.

10. Anordnung nach einem der Ansprüche 1 bis 9, **dadurch gekennzeichnet, dass** eine Datenübertragung von der Anzeigeeinheit (17) zu der Informationsquelle (20) vorgesehen ist.

11. Operationsmikroskop nach einem der Ansprüche 1 bis 10, **dadurch gekennzeichnet, dass** eine Einspiegelungseinheit (16) vorgesehen ist und dass Informationen von der Anzeigeeinheit (17) in der Einspiegelungseinheit (16) eingeblendet werden können.

12. Operationsmikroskop nach einem der Ansprüche 1 bis 11, **dadurch gekennzeichnet, dass** die Anzeigeeinheit (17) mit einer sterilen Abdeckung umgeben ist und dass der Bildschirm (18) von der sterilen Abdeckung ausgenommen ist.
